(19) Europäisches Patentamt
European Patent Office
Office européen des brevets

(11) Publication number: **0 405 748 A1**

# EUROPEAN PATENT APPLICATION

(21) Application number: **90305689.3**

(22) Date of filing: **24.05.90**

(51) Int. Cl.[5]: **C07F 9/38**, C07F 9/6574, A61K 31/66

(30) Priority: **30.05.89 GB 8912348**
**30.05.89 GB 8912349**

(43) Date of publication of application:
**02.01.91 Bulletin 91/01**

(84) Designated Contracting States:
**AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Harnden, Michael Raymond,**
**Beecham Pharmaceuticals**
**Great Burgh, Yew Tree Bottom Road**
**Epsom, Surrey KT18 5XQ(GB)**
Inventor: **Duckworth, David Malcolm,**
**Beecham Pharmaceuticals**
**Great Burgh, Yew Tree Bottom Road**
**Epsom, Surrey KT18 5XQ(GB)**

(74) Representative: **Jones, Pauline et al**
**SmithKline Beecham, Corporate Patents,**
**Great Burgh, Yew Tree Bottom Road**
**Epsom, Surrey KT18 5XQ(GB)**

## (54) Phosphonoderivative of purine with antiviral activity.

(57) The (R)-isomer or the (S)-isomer of a compound of formula (IA), optionally in admixture with up to 45% of the mixture by weight of the corresponding (S)-isomer or the (R)-isomer respectively:

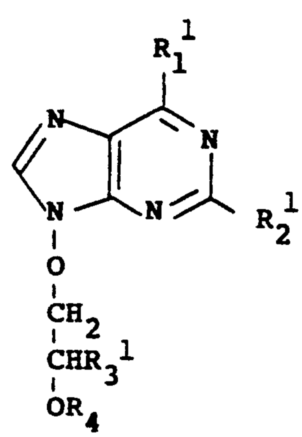

(IA)

wherein
$R_1{}^1$ is hydroxy or amino;
$R_2{}^1$ is amino or hydrogen;
$R_3{}^1$ is hydroxymethyl or acyloxymethyl; and
$R_4$ is a group of formula:

EP 0 405 748 A1

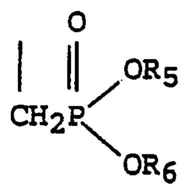

wherein
$R_5$ and $R_6$ are independently selected from hydrogen, $C_{1-6}$ alkyl and optionally substituted phenyl; or
$R_3$ and $R_4$ together are:

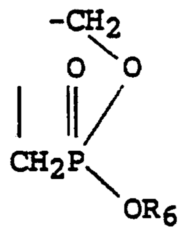

wherein
$R_6$ is as defined above;
having antiviral activity, a process for their preparation and their use in the treatment of viral infections or neoplastic diseases.

## ACTIVE COMPOUNDS

The present invention relates to one isomeric form of chemical compounds having antiviral activity, to processes for the preparation of such isomers and to their use as pharmaceuticals.

EP-A-319228 (Beecham Group p.l.c.), the subject matter of which is incorporated herein by reference, discloses antiviral compounds of formula (I) and pharmaceutically acceptable salts thereof:

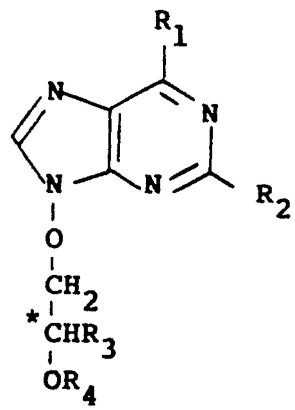

(I)

wherein
$R_1$ is hydroxy, amino, chloro or $OR_7$
wherein
$R_7$ is $C_{1-6}$ alkyl, phenyl or phenyl $C_{1-2}$ alkyl either of which phenyl moieties may be substituted by one or two substituents selected from halo, $C_{1-4}$ alkyl or $C_{1-4}$ alkoxy;
$R_2$ is amino or, when $R_1$ is hydroxy or amino, $R_2$ may also be hydrogen;
$R_3$ is, hydrogen, hydroxymethyl or acyloxymethyl;
$R_4$ is a group of formula:

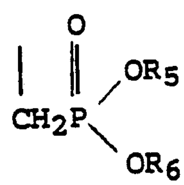

wherein
$R_5$ and $R_6$ are independently selected from hydrogen, $C_{1-6}$ alkyl and optionally substituted phenyl; or
$R_3$ and $R_4$ together are:

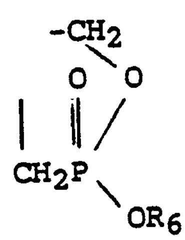

wherein
$R_6$ is as defined above.

Examples 6-10, 15 and 16 describe the (R,S) racemic mixture of the compounds wherein $R_3$ is other than hydrogen (asymmetric carbon atom denoted by "*" in formula (I)). The pure (R)- and (S)-isomers have now been prepared and have been found to have antiviral activity.

Accordingly, the present invention provides the (R)-isomer or the (S)-isomer of a compound of formula (IA), optionally in admixture with up to 45% of the mixture by weight of the corresponding (S)-isomer or the (R)-isomer respectively:

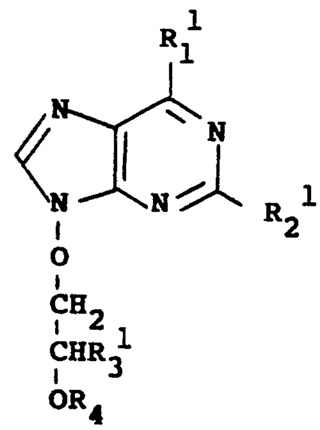

(IA)

wherein
$R_1{}^1$ is hydroxy or amino;
$R_2{}^1$ is amino or hydrogen;
$R_3{}^1$ is hydroxymethyl or acyloxymethyl; and
$R_4$ is defined in relation to formula (I).

When $R_1{}^1$ is hydroxy and $R_2{}^1$ is amino, the compound of formula (IA) is a guanine derivative;
When $R_1{}^1$ is amino and $R_2{}^1$ is hydrogen, the compound of formula (IA) is an adenine derivative;
When $R_1{}^1$ is hydroxy and $R_2{}^1$ is hydrogen, the compound of formula (IA) is a hypoxanthine derivative; and
When $R_1{}^1$ and $R_2{}^1$ are both amino groups, the compound of formula (IA) is a 2,6-diaminopurine derivative.

Often, the compound of formula (IA) is a guanine or adenine derivative.

Suitable examples of the acyl group in $R_3{}^1$ when acyloxymethyl, include carboxylic acyl, such as $C_{1-7}$ alkanoyl and benzoyl optionally substituted in the phenyl ring as defined below for $R_5/R_6$. Preferred acyl groups include acetyl, propionyl, butyryl, heptanoyl and hexanoyl.

Suitable examples of $R_5$ and $R_6$ include hydrogen, methyl, ethyl, n - and iso-propyl, n , sec -, iso - and tert -butyl, and phenyl optionally substituted by one, two or three groups or atoms selected from halogen, such as fluoro, chloro, bromo, and $C_{1-4}$ alkyl or $C_{1-4}$ alkoxy wherein the alkyl moiety is selected from those listed for $R_5/R_6$ above.

Examples of pharmaceutically acceptable salts of the compound of formula (I) are acid addition salts formed with a pharmaceutically acceptable acid such as hydrochloric acid, orthophosphoric acid and sulphuric acid. Pharmaceutically acceptable salts also include those formed with organic bases, preferably with amines, such as ethanolamines or diamines; and alkali metals, such as sodium and potassium.

As the compound of formula (IA) contains a phosphonate group, suitable salts include metal salts, such as alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

The compounds of formula (IA) including their alkali metal salts may form solvates such as hydrates and these are included wherever a compound of formula (IA) or a salt thereof is herein referred to.

It will be appreciated that, when $R_1{}^1$ is hydroxy in formula (IA) the compound exists in the predominant tautomeric form of structure (IB):

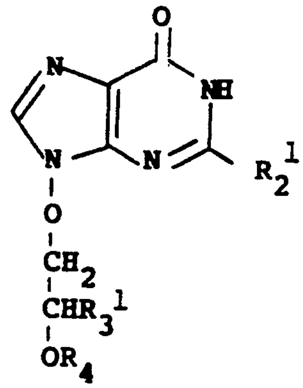

(IB)

Preferably, the (R)-isomer or the (S)-isomer of a compound of formula (IA) or a derivative thereof as defined is in a form containing from 0 to 40%, 0 to 30%, 0 to 20% or 0 to 10% of the corresponding (S)-isomer or the (R)-isomer respectively. More preferably, the (R)-isomer or the (S)-isomer is in a form containing 0 to 5% of the corresponding (S)-isomer or the (R)-isomer respectively. Most preferably, the (R)-isomer or the (S)-isomer is in a form containing 0% or no detectable amount of the corresponding (S)-isomer or the (R)-isomer respectively. All percentages hereinbefore are percentages of the mixture by weight. The presence of the undesired isomer may, for example, be routinely detected by the comparison of the optical rotation of a sample of the desired isomeric mixture with that of a pure sample of the desired isomer, or by the $^1$H nmr spectrum of a sample of the isomeric mixture in the presence of a chiral shift reagent or chiral solvating agent.

The invention also provides a process for the preparation of a compound of formula (IA), or a pharmaceutically acceptable salt thereof, which process comprises either

i) imidazole ring closure of a compound of formula (II):

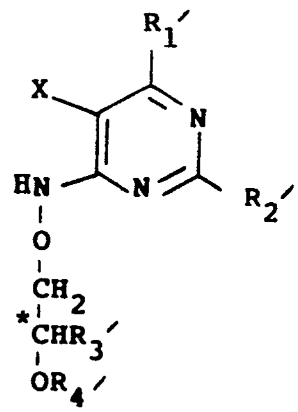

(II)

wherein X is a group capable of cyclising to form an imidazole ring, such as amino or an amino derivative, for example, formylamino; or

ii) pyrimidine ring closure of a compound of formula (III):

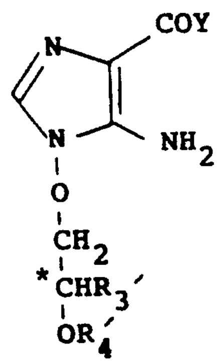

(III)

wherein Y is amino or $C_{1-6}$ alkoxy, with a condensing agent capable of cyclising to form a pyrimidine ring having a 2-$R_2'$ substituent, to give a compound of formula (I) wherein $R_1$ is hydroxy and $R_2$ is amino; or

iii) condensing a compound of formula (IV):

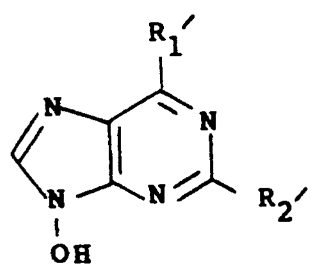

(IV)

with a side chain intermediate of formula (V):

$ZCH_2$- $\overset{*}{C}HR_3'OR_4'$ (V)

wherein Z is a leaving group and the carbon atom marked "*" in formulae (II), (III) and (V) is 55% or more in the configuration resulting in the (R)-isomer or the (S)-isomer of a compound of formula (IA); and wherein, in formulae (II) to (V), $R_1'$, $R_2'$, $R_3'$, $R_4'$ are $R_1^1$, $R_2^1$, $R_3^1$ and $R_4$ respectively, or groups or atoms convertible thereto; and thereafter, when desired or necessary, converting $R_1'$, $R_2'$, $R_3'$ and/or $R_4'$, when other than $R_1^1$, $R_2^1$, $R_3^1$ and/or $R_4$ to $R_1^1$, $R_2^1$, $R_3^1$ and/or $R_4$ respectively, and/or converting $R_1'$, $R_2'$, $R_3'$ and/or $R_4'$ when $R_1^1$, $R_2^1$, $R_3^1$ and/or $R_4$, to other $R_1^1$, $R_2^1$, $R_3^1$ and/or $R_4$.

Process i) may be carried out, preferably when X is formyl, using a cyclisation condensing agent, such as diethoxymethyl acetate or triethyl orthoformate, or by fusion.

Process ii) is preferably carried out in accordance with the methods described in EP-A-242482, the subject matter of which is incorporated herein by reference.

Process iii) may be carried out with suitable values for Z including hydroxy and halo, such as chloro, bromo and iodo, preferably iodo; or other groups readily displaceable by nucleophiles, such as mesyloxy or tosyloxy. The reaction preferably takes place in an inert solvent, such as dimethylformamide at 0-50°C, preferably ambient temperature. When Z is hydroxy, the reaction takes place in the presence of a dehydrating catalyst, such as diethyl azodicarboxylate in the presence of triphenylphosphine. When Z is halo, the reaction preferably takes place in the presence of a base, such as potassium carbonate.

Examples of conversions of variable groups are as follows:

$R_1'$-$R_1^1$

a) An $R_1^1$ hydroxy group may be converted to $R_1'$ is chloro, by chlorination using a reagent such as phosphorus oxychloride, preferably in the presence of tetraethylammonium chloride and dimethylaniline (as acid acceptor) in $CH_3CN$ at reflux temperatures, according to the method described by M.J. Robins

and B. Ozanski, Can. J. Chem, 59 , 2601 (1981).

b) An $R_1'$ chloro group may be converted to $R_1^1$ is hydroxy by hydrolysis using aqueous mineral acid, such as hydrochloric acid, or more preferably, using an organic acid, such as formic acid at elevated temperature, suitably 70-150°C, preferably around 100°C.

c) An $R_1'$ chloro group may be converted to $R_1^1$ is amino by treatment with ammonia in a lower alkanol, such as ethanol or methanol in an autoclave at 100°C for a period of about 7 hours, or alternatively, by treatment with sodium azide in dimethylformamide (forming an $R_1^1$ is $N_3$ intermediate), followed by reduction with ammonium formate/palladium or charcoal, in methanol.

d) An $R_1'$ alkoxy group, such as methoxy, may be converted to $R_1^1$ hydroxy by the methods of D.R. Haines, J. Med. Chem. 1987, 30 , 943 and K.K. Ogilvie and H.R. Hanna, Can. J. Chem. 1984, 62 , 2702.

e) An $R_1'$ protected amino group, such as tritylamino, may be converted to amino, by treatment with aqueous acetic acid, preferably 80% acetic acid at elevated temperature, around 80°C. $R_1'$ may also be phthalimido, which may be converted to amino by treatment with methyl hydrazine or hydrazine in an inert solvent, such as dichloromethane, at ambient temperature.

$R_2'$-$R_2^1$

a) $R_2'$ may be protected amino, such as formylamino, which may be converted to $R_2^1$ is amino by hydrolysis.

$R_3'$-$R_3^1$

a) $R_3^1$ hydroxymethyl may be converted to $R_3^1$ acyloxymethyl by conventional acylation procedures.

b) $R_3'$ may be protected hydroxymethyl, which may be converted to $R_3^1$ hydroxymethyl by conventional deprotection methods.

Suitable examples of protecting groups and their removal, are as described in EP-A-242482 and 319228. A particularly suitable protecting group also includes the t-butyldiphenylsilyl group, removable by treatment with aqueous acid, such as trifluoracetic acid or mineral acid, at ambient temperature.

$R_4'$-$R_4$

a) When $R_5$ and $R_6$ in $R_4$ are other than hydrogen, they may be converted to $R_5$ and $R_6$ are hydrogen, using a deesterifying reagent, such as trimethylsilyl bromide in an aprotic solvent such as dichloromethane or dimethylformamide at ambient temperature, as described by C.E. McKenna et. al., J.C.S. Chem. Comm., 1979, 739.

Selective conversion of one $R_5$ and $R_6$ to hydrogen, may be achieved by treatment with hydroxide ion, as described by Rabinowitz, JACS 1960, 82 , 4564.

b) $R_4'$ may be hydrogen, which may be converted to $R_4$, by treatment with $QR_4$ wherein Q is a leaving group and $R_4$ is as defined. Q is preferably a tosyloxy group. Conditions for this reaction are i) preliminary formation of an alkoxide using a base, such as sodium hydride, in an aprotic solvent for example dimethylformamide ii) reaction with $QR_4$ at around ambient temperature. The reaction is as described A. Holy et. al., Collect. Czech. Chem. Comm., 1982, 47 , 3447.

In this case, $R_5$ and $R_6$ are preferably other than hydrogen.

c) $R_4'$ may be hydrogen, which may be converted, when $R_3$ is hydroxymethyl, to $R_4$ is $CH_2PO(OH)$-($OR_5$), by treatment with $ClCH_2PCl_2$ followed by treatment with a base, followed by $OR_5^-$, according to the method described by A. Holy et. al. Czech. Chem. Comm. 1985, 50 , 1507; ibid 1987, 52 , 2775.

d) When $R_5$ is hydrogen, and $R_3^1$ is hydroxymethyl, they may be converted to a compound wherein $R_3^1$ and $R_4$ together form a cyclic phosphonate as defined in formula (I), by reaction with N,N-dicyclohexyl-4-morpholinocarboxamidine in the presence of a dehydrating catalyst, such as N,N-dicyclohexylcarbodiimide.

It will be appreciated that the above conversions may take place in any desired or necessary order, having regard to the final desired compound of formula (I).

Intermediates of formula (II) may be prepared from a corresponding compound of formula (VI):

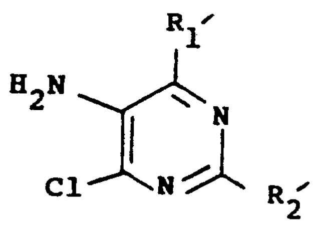

(VI)

and via intermediates of formula (V) wherein Z is OH, as hereinbefore defined, according to the methods described in EP-A-242482 i.e. by converting the compound of formula (V) wherein Z is OH to the phthalimidooxy derivative followed by reaction with methylhydrazine.

The compound of formula (VI) wherein $R_1'$ is chloro and $R_2'$ is amino, is a known compound as described by Temple et. al., J. Org. Chem., 40 (21), 3141, 1975.

The compound of formula (VI) wherein $R_1'$ is chloro and $R_2'$ is hydrogen is a commercially available compound.

Intermediates of formula (III) may be prepared according to the methods described in EP-A-242482.

Compounds of the formula (IV) are prepared from compounds of formula (VI) wherein the 5-amino group is formylated, by reaction with $R_8ONH_2$ wherein $R_8$ is a protecting group, to give a compound of formula (VII):

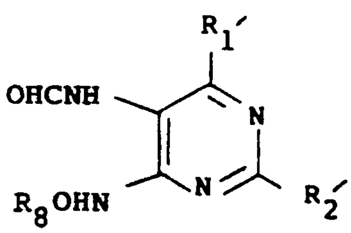

(VII)

which may be cyclised with diethoxymethyl acetate, to give a compound of formula (IV) wherein the OH group is protected. Suitable values for $R_8$ include benzyl, removable by hydrogenation, and the tetrahydropyran-2-yl group removable by treatment with 80% acetic acid, at ambient temperature.

Intermediates of the formula (V) wherein Z is hydroxy are known compounds or are prepared by analogous methods to those used for structurally similar known compounds.

It will be appreciated that, synthesis of the intermediate of formula (V) wherein Z is hydroxy will involve selective deprotection of an intermediate wherein Z is protected hydroxy and $R_3$ is protected hydroxymethyl, for example, as shown in the following scheme, when the (R)-isomer is desired.

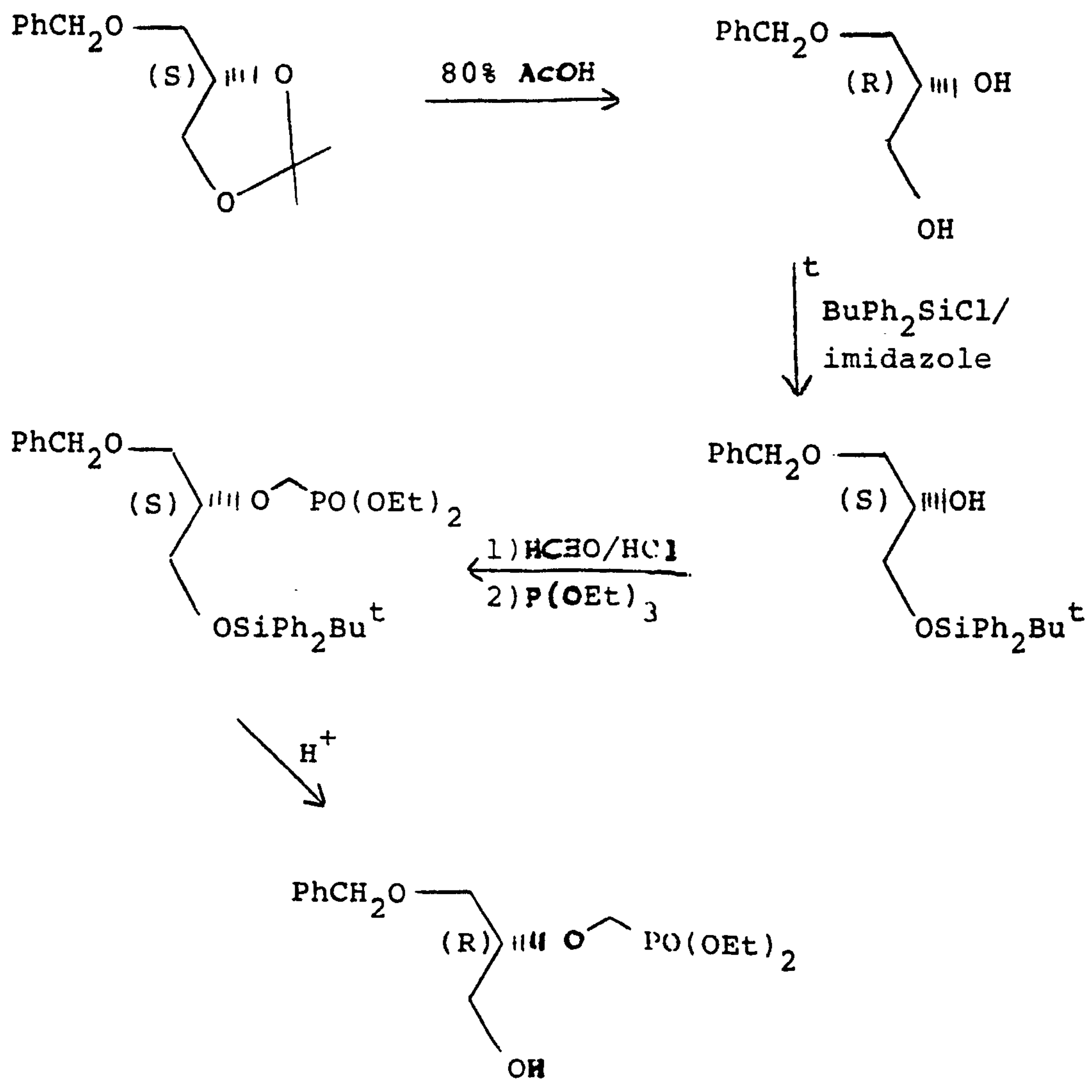


N.B. All depicted configurations are reversed when the (S)-isomer is desired.

Intermediates of formulae (II), (III) and (V) but wherein Z is replaced by an aminooxy group, and wherein $R_4'$ is $R_4$ as defined in formula (I), are believed to be novel and form an aspect of the invention.

Pharmaceutically acceptable salts may be prepared in conventional manner, for example, in the case of acid addition salts, by reaction with the appropriate organic or inorganic acid.

It will be appreciated that the invention provides a process for the preparation of a compound of formula (IA) wherein $R_3^1$ is hydroxymethyl which process comprises the deprotection of a compound of formula (IA) wherein an OH group in $R_3^1$ is in protected form. Preferred methods for deprotection are as hereinbefore described.

The invention also provides a process for the preparation of a compound of formula (IA) wherein $R_5$ and $R_6$ are both hydrogen, which process comprises the deesterification of a corresponding compound of formula (IA) wherein $R_5$ and $R_6$ are the same alkyl or optionally substituted phenyl group.

The present invention also provides a process for the preparation of the (R)-isomer or the (S)-isomer of a compound of formula (IA) or a pharmaceutically acceptable salt thereof, optionally in admixture with up to 45% of the mixture by weight of the corresponding (S)-isomer or the (R)-isomer respectively, which process comprises resolution of a mixture of (R)- and (S)-isomers of a compound of formula (IA) to the extent that the resolution fraction which predominantly contains (R)-isomer or the (S)-isomer optionally contains up to 45% of that resolution fraction by weight of the corresponding (S)-isomer or the (R)-isomer respectively; and optionally forming a pharmaceutically acceptable salt thereof.

The term 'resolution' is used herein in the conventional practical sense used in the art to include partial resolution, that is, the separation of a mixture of enantiomers of a compound (in any ratio) into two fractions, one of which is enriched in one enantiomer relative to the initial mixture.

Resolution of the mixture of (R)- and (S)- isomers of a compound of formula (IA) may be effected conventionally by derivatising the mixture with a chiral derivatising agent, to form a mixture of diastereomers of a derivative of the compound of formula (IA). The components of the mixture may then be separated conventionally, for example by fractional crystallisation. Separation may be complete, or partial, such that a given separated fraction comprises up to 45% of the total fraction by weight of the diastereomeric derivative of the (S)-isomer or the (R)-isomer. Subsequently, the diastereomeric derivative of the desired (R)-isomer or the (S)-isomer respectively, optionally in admixture with the diastereomeric derivative of the (S)-isomer or the (R)-isomer respectively, is converted to the desired isomer of the compound of formula (IA), optionally in admixture with up to 45% of the other isomer.

The derivatisation for the process of the present invention is preferably effected on a mixture of (R)- and (S)-isomers of a compound of formula (IA) by reacting such a mixture of isomers with an optically active reagent suitable for resolving alcohols. Such reagents include optically active acids or acid derivatives, such as acid chlorides, acid anhydrides or isocyanates.

Salts and derivatives may be prepared as hereinbefore described.

A racemic mixture of isomers may be prepared in accordance with the method described in EP-A-319228.

The preferred process for the preparation of a pure isomer of formula (IA) is, however, via chiral intermediates of formula (V).

The compounds of the invention are of potential use in the treatment of infections caused by viruses, especially herpesviruses such as herpes simplex type 1, herpes simplex type 2 and varicella-zoster virus; Epstein-Barr virus and cytomegalovirus; and retroviruses, including lentiviruses such as visna virus and human immunodeficiency virus (strains 1 and 2).

The compounds may also be inhibitors of tumorogenic viruses and/or of potential use in the treatment of neoplastic diseases, i.e. cancer.

Compounds of the invention may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that may be administered in a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

No unacceptable toxicological effects are indicated at the above described dosage levels.

The invention also provides a method of treating viral infections in a human or non-human animal, which comprises administering to the animal an effective, non-toxic amount of a compound of the invention or a pharmaceutically acceptable salt thereof.

The invention also provides a compound of the invention or a pharmaceutically acceptable salt thereof for use as an active therapeutic substance, in particular for the treatment of viral infections.

The compounds of the invention are also believed to exhibit a synergistic antiviral effect in conjunction with interferons; and combination products comprising these two components for sequential or concomitant administration, by the same or different routes, are therefore within the ambit of the present invention.

The following examples illustrate the invention; the following descriptions illustrate the preparation of intermediates. Examples 1 to 3 are (R)-isomer examples and Examples 4 to 6 are (S)-isomer examples.

It will be appreciated that, when $R_3$ and $R_4$ are joined in formula (I), such as in Examples 2 and 5, the designation of absolute sterochemistry is reversed, in accordance with the sterochemistry priority rules.

Description 1

a) (S)-1-Benzyloxy-3-(t-butyldiphenylsilyloxy)propan-2-ol

To a solution of (R)-3-benzyloxypropane-1,2-diol (prepared from (S)-4-benzylymethyl-2,2-dimethyl-1,3-dioxolane (commercially available from FLUKA) by acid hydrolysis using 80% aqueous acetic acid), (8.3g, 47.2mmol) in dry THF (70ml) was added imidazole (6.42g, 94.4mmol). After 2 minutes, t-butyldiphenylsilyl chloride (12.28ml, 47.2mmol) was added. A white precipitate formed immediately. After 2 hours, the reaction mixture was filtered and the filtrate evaporated. The residue was partitioned between ethyl acetate and water, the organic layer separated, washed with saturated brine, dried ($MgSO_4$), filtered and evaporated to give a colourless oil. Chromatography of the oil on silica gel (dichloromethane as eluant) afforded the title compound as a colourless oil (17.72g, 90%).

IR: $\nu_{max}$ (film) 3580, 3070, 2940, 2860, 1595, 1475, 1455, 1430, 1395, 1365, 1345, 1310, 1270, 1265, 1240, 1110, 1030, 1010, 1000, 960, 940, 828, 810, 745, 710cm$^{-1}$. $^1$H NMR: $\delta_H$ [$(CD_3)_2SO$] 0.97 (9H, s, t-Bu), 3.4-3.85 (5H, m, $CH_2CHCH_2$), 4.50 (2H, s, OC H $_2$Ph), 4.84 (1H, d, J = 5.3Hz, $D_2O$ exchangeable OH), 7.25-7.70 (15H, m, aromatic H). Found: C, 74.27, H, 7.71%. $C_{26}H_{32}O_3Si$ requires: C, 74.24; H, 7.67%. m/z: ($NH_3$ C.I.) 438 ($MNH_4^+$, 8%). [α]D$^{25}$ ($CHCl_3$) -1.7° (C = 0.94).

b) Diethyl (S)-[2-benzyloxy-1-(t-butyldiphenylsilyloxymethyl)ethoxy]methylphosphonate

Dry HCl gas was bubbled through a solution of (S)-1-benzyloxy-3-(t-butyldiphenylsilyloxy)propan-2-ol (4.206g, 0.01mmol) and paraformaldehyde (0.3g, 0.01mmol) in dry dichloromethane (20ml) containing anhydrous calcium chloride (7.5g), for 1 hour. Any remaining traces of water were removed by drying over magnesium sulphate. The solution was filtered and evaporated to dryness to give a colourless oil. Triethyl phosphite (1.71ml, 0.01mmol) was added and the solution was heated at 140°C for 5 hours, then allowed to cool to ambient temperature. The reaction product was chromatographed on silica gel (dichloromethane: methanol, 99:1 as eluant) to give the title compound as a colourless oil (2.25g, 40%).

IR: $\nu_{max}$ (film) 3060, 2960, 2930, 2900, 2850, 1475, 1455, 1430, 1390, 1365, 1260, 1115, 1055, 1030, 960, 820, 805, 740, 710cm$^{-1}$. $^1$H NMR: $\delta_H$ ($CDCl_3$) 1.03 (9H, s, t-Bu), 1.28 (2x3H, 2xt, J = 7Hz, ($OCH_2C$ H $_3$)$_2$), 3.5-3.85 (5H, m, $CH_2CHCH_2$), 3.96 (2H, d, J = 8.9Hz, $OCH_2P$), 4.05-4.25 (4H, m, (OC H $_2CH_3$)$_2$), 4.52 (2H, s, OC H $_2$Ph), 7.25-7.75 (15H, m, aromatic H). Found: C, 65.43; H, 7.70%. $C_{31}H_{43}O_6PSi$ requires: C, 65.24; H, 7.59%. m/z: ($NH_3$ C.I.) 588 ($MNH_4^+$, 35%), 571 ($MH^+$, 100%). [α]D$^{25}$ ($CHCl_3$) -6.90° (C = 1.06).

Description 2 [(R)-isomers]

Diethyl (S)-[1-(t-butyldiphenylsilyloxymethyl)-2-hydroxyethoxy]methylphosphonate

To a solution of diethyl (S)-[2-benzyloxy-1-(t-butyldiphenylsilyloxymethyl)ethoxy]methylphosphonate (1.90g, 3.3mmol) in ethanol (20ml) containing a trace of methanol/HCl (10 drops), was added 10%Pd-C (1.2g). The mixture was shaken under an atmosphere of hydrogen at ambient temperature and atmospheric pressure until hydrogen uptake was complete and t.l.c. ($SiO_2$, $CH_2Cl_2$-MeOH, 95:5) indicated no starting material remained. The reaction mixture was filtered, the filtrate evaporated and the residue obtained chromatographed on silica gel (dichloromethane-methanol, 95:5 as eluant) to give the title compound as a colourless oil (1.35g, 84%).

IR: $\nu_{max}$ (film) 3420, 3080, 3060, 2945, 2870, 1595, 1475, 1430, 1395, 1365, 1245, 1115, 1060, 1030, 975, 825, 745, 715cm$^{-1}$. $^1$H NMR: $\delta_H$ [$(CD_3)_2SO$] 1.0 (9H, s, t-Bu), 1.20 (2x3H, 2xt, J = 7.1Hz, ($OCH_2C$ H $_3$)$_2$), 3.45-3.60 (3H, m, $CH_2CH$), 3.65-3.80 (2H, m, $CH_2$), 3.90-4.15 (6H, m, $OCH_2P$,(OC H $_2CH_3$)$_2$), 4.65 (1H, t, J = 5.2Hz, $D_2O$ exchangeable OH), 7.30-7.75 (10H, m, aromatic H). Found: C, 60.11; H, 7.87%.$C_{24}H_{37}O_6PSi$

requires: C, 59.97; H, 7.76%. m/z: ($NH_3$ C.I.) 498 ($MNH_4^+$, 15%), 481 ($MH^+$, 100%). [α]D[25] ($CHCl_3$) -19.0° - (C = 0.4).

Description 3 [(S)-isomers]

Diethyl (R)-[2-benzyloxy-1-(hydroxymethyl)ethoxy]methylphosphonate

A suspension of diethyl (S)-[2-benzyloxy-1-(t-butyldiphenylsilyloxymethyl)ethoxy]methylphosphonate (3.09g, 5.4mmol) in trifluoroacetic acid/water 2:1 (12ml) was stirred at ambient temperature for 1 hour. The solution obtained was extracted with hexane (2x10ml) and the aqueous containing phase evaporated to dryness. The residue obtained was treated with ethanolic ammonia (5ml) for 5 minutes at ambient temperature, the solvent evaporated and the residue chromatographed on silica gel using dichloromethane/methanol 95:5 as eluant. The title compound was obtained as a colourless oil (1.35g, 75%).

IR: $\nu_{max}$ (film) 3380, 3060, 3030, 2980, 2910, 2860, 1495, 1480, 1450, 1390, 1370, 1250, 1160, 1100, 1075, 975, 910, 890, 820, 780, 740, 700cm$^{-1}$. $^1$H NMR: $\delta_H$ [$(CD_3)_2SO$] 1.21, 1.22 (2x3H, 2xt, J = 7 Hz, ($OCH_2CH_3$)$_2$), 3.4-3.65 (5H, m, $CH_2$, CH,$CH_2$), 3.94 (2H, d, J = 8.5 Hz, $OCH_2P$), 3.97-4.1 (4H, m, ($OCH_2CH_3$)$_2$), 4.5 (2H, s, $CH_2Ph$), 4.67 (1H, t, J = 5.5 Hz, $D_2O$ exchangeable OH), 7.20-7.40 (5H, m, Ph). Found: C, 53.20; H, 7.72%. $C_{15}H_{25}O_6P$ 0.33 $H_2O$ requires: C, 53.25; H, 7.64%. m/z: observed 332.1383. $C_{15}H_{25}O_6P$ requires 332.1387. [α]D[25] ($CHCl_3$) +16.7° (c 1.06).

Example 1

(R)-9-[3-Hydroxy-2-(phosphonomethoxy)propoxy]quanine

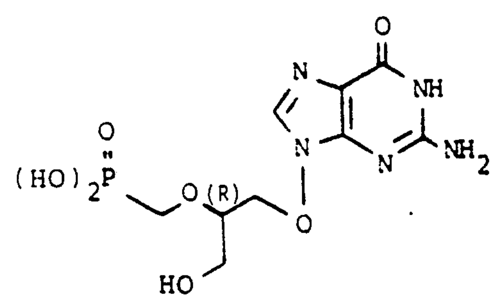

a) To a stirred mixture of diethyl (S)-[1-(t-butyldiphenylsilyloxymethyl)-2-hydroxyethoxy]-methylphosphonate (540mg, 1.12mmol), triphenylphosphine (440mg, 1.68mmol) and 2-[bis-(t-butoxycarbonyl)amino]-9-hydroxy-6-methoxypurine (prepared as described in EP-A-319228) (428mg, 1.12mmol) in dry THF (20ml), cooled in ice and under a nitrogen atmosphere, was added dropwise diethyl azodicarboxylate (0.27ml, 1.68mmol). Within five minutes complete dissolution had occurred and the ice bath was removed. After 1 hour, the solution was evaporated to dryness and the residue obtained chromatographed on silica gel (initial eluant hexane: ethyl acetate 2:1, then hexane:ethyl acetate in the proportions 1:1, 1:2, 1:3) to yield (S)-2-[bis-(t-butoxycarbonyl)amino]-9-[3-(t-butyldiphenylsilyloxy)-2-(diethoxyphosphorylmethoxy)propoxy]-6-methoxypurine as a colourless glass (310mg, 32%). $^1$H NMR: $\delta_H$ [$(CD_3)_2SO$] 0.96 (9H, s, t-Bu), 1.18, 1.19 (2x3H, 2xt, J = 7Hz, ($OCH_2CH_3$)$_2$), 1.37 (18H, br.s, 2xt-Bu), 3.7-4.15 (12H, m, $CHCH_2$, ($OCH_2CH_3$)$_2$, $OCH_2P$, $OCH_3$), 4.45-4.70 (2H, m, $NOCH_2$), 7.25-7.75 (10H, m, 2xPh), 8.75 (1H, s, H-8). m/z: (FAB +ve ion, thioglycerol/HCl) 844 ($MH^+$, 4%), 644 (100%).

b) A solution of (S)-2-[bis-(t-butyloxycarbonyl)amino]-9-[3-(t-butyldiphenylsilyloxy)-2-(diethoxyphosphorylmethoxy)propoxy]-6-methoxypurine (300mg, 0.36mmol) in 67% aqueous trifluoroacetic acid (3ml) was kept at ambient temperature for 3 hours. The solution was washed with hexane (3x10ml) and the aqueous phase evaporated to dryness. The residue obtained was chromatographed on silica gel (dichloromethane: methanol 95:5 as eluant, then 90:10) to give (R)-2-amino-9-[2-(diethoxyphosphorylmethoxy)-3-hydroxypropoxy]-6-methoxypurine as a pale yellow glass (101mg, 70%).

$^1H$ NMR: $\delta_H$ [$(CD_3)_2SO$] 1.23 (6H, t, J = 7.1Hz, ($OCH_2CH_3)_2$), 3.5-3.65 (2H, m, $CH_2$), 3.70-3.80 (1H, m, CH), 3.96 (3H, s, $OCH_3$), 3.98-4.10 (6H, m, ($OCH_2CH_3)_2$, $OCH_2P$), 4.31 (1H, dd, J = 11.27Hz, J = 6.87Hz, $NOCH_B$), 4.45 (1H, dd, J = 11.27Hz, J = 3.02Hz, $NOCH_A$), 4.85 (1H, t, J = 5.50Hz, $D_2O$ exchangeable OH), 6.60 (2H, s, $D_2O$ exchangeable $NH_2$), 8.14 (1H, s, H-8). m/z: (FAB +ve ion, thioglycerol) 406 ($MH^+$, 100%).

c) To a solution of (R)-2-amino-9-[2-(diethoxyphosphorylmethoxy)-3-hydroxypropoxy)-6-methoxypurine (100mg, 0.25mmol) in dry dimethylformamide (1ml) was added trimethylsilyl bromide (0.5ml, 3.8mmol) and the solution stirred at ambient temperature for two hours. The solvent was removed under reduced pressure and the residue co-evaporated with acetone-water, 1:1 (x2). The solid obtained was crystallised from hot water to give the title compound as a cream solid (16mg, 20%). $^1H$ NMR: $\delta_H$ [$(CD_3)_2SO$] 3.52 (2H, m, $CH_2OH$), 3.60-3.80 (3H, m, $OCH_2P$, CH), 4.25 (1H, dd, J = 11.9Hz, J = 7.1Hz, $NOCH_B$), 4.40 (1H, dd, J = 11.9Hz, J = 3.3Hz, $NOCH_A$), 6.60 (2H, br.s, $D_2O$ exchangeable $NH_2$), 8.00 (1H, s, H-8), 10.65 (1H, br.s, $D_2O$ exchangeable NH), 2.6-4.1 (3H, broad, $D_2O$ exchangeable OH, $PO(OH)_2$). m/z: (FAB +ve ion, thioglycerol) 336 ($MH^+$, 28%).

## Example 2

(S)-9-[(2-Hydroxy-2-oxo-1,4,2-dioxaphosphorinan-5-yl)methoxy]quanine

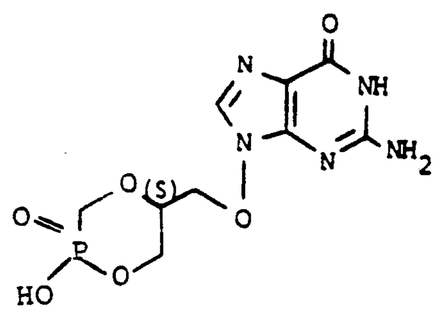

A solution of dicyclohexylcarbodiimide (0.615g, 3.0mmol) in t-butanol (15ml) was added dropwise over 30 minutes to a solution of (R)-9-[3-hydroxy-2-(phosphonomethoxy)propoxy]guanine (0.20g, 0.6mmol) and N,N-dicyclohexyl-4-morpholinocarboxamidine (0.175g, 0.6mmol) in 50% aqueous t-butanol (30ml). The reaction mixture was heated under reflux for $5\frac{1}{2}$ hours, allowed to cool to ambient temperature and evaporated to dryness. Water was added to the residue and the resulting mixture filtered through a glass fibre filter paper. The filtrate was evaporated to dryness and the solid obtained purified on DEAE-Sephadex ($HCO_3^-$ form) eluting with a linear gradient of aqueous triethylammonium bicarbonate (pH 7.5) from 0.001M to 0.25M. Relevant fractions were combined and evaporated to dryness co-evaporating with ethanol/water 3:1 (x3) to remove all traces of triethylamine. The product was converted into the sodium salt using DOWEX 50W-X8 ($Na^+$-form). The title compound was obtained as a white powder (0.16g, 79%) after lyphilisation. UV: $\lambda_{max}$ ($H_2O$) 253 ($\epsilon$ 11,180)nm. IR: $\nu_{max}$ (KBr) 3411, 3400, 3144, 2759, 1696, 1617, 1591, 1476, 1380, 1330, 1239, 1209, 1174, 1081, 1045, 1010, 960, 815, 796 $cm^{-1}$. $^1H$ NMR: $\delta_H$ [$(CD_3)_2SO$] 3.40 (1H, dd, J = 12,9 Hz, J = 3.3 Hz, $OCH_BP$) 3.50 (1H, dd, J = 12.9 Hz, J = 8 Hz, $OCH_AP$), 3.74 (1H, m, CH), 3.90 (1H, m, $CH_BOP$), 4.0-4.3 (3H, m, $CH_AOP$, $NOCH_2$), 6.75 (2H, br.s, $D_2O$ exchangeable $NH_2$), 7.90 (1H, s, H-8), 10.81 (1H, br.s, $D_2O$ exchangeable NH). m/z: (FAB, thioglycerol matrix) 340 ($MH^+$). $[\alpha]D^{25}$ ($H_2O$) - 38.7° (c = 0.93).

## Example 3

(R)-9-[3-Hydroxy-2-(phosphonomethoxy)propoxy]adenine

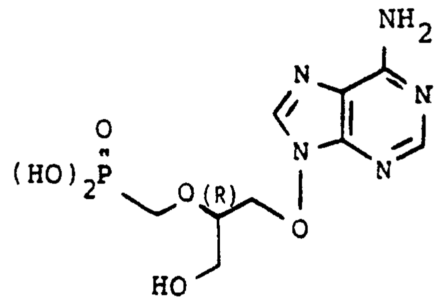

a) To a stirred mixture of diethyl (S)-[1-(t-butyldiphenylsilyloxymethyl)-2-hydroxyethoxy]-methylphosphonate (1.07g, 2.2mmol), triphenylphosphine (0.7g, 2.7mmol) and 9-hydroxy-6-phthalimidopurine (prepared as described in EP-A-319228) (0.63g, 2.2mmol) in dry THF (20ml), cooled in ice and under a nitrogen atmosphere, was added dropwise diethyl azodicarboxylate (0.42ml, 2.7mmol). The resulting solution was stirred overnight at ambient temperature, evaporated to dryness and the residue obtained chromatographed on silica gel using hexane/ethyl acetate 3:1 as eluant. The eluant was changed as follows: hexane/ethyl acetate 1:1, hexane/ethyl acetate 1:2 and finally to ethyl acetate to give (S)-9-[3-(t-butyldiphenylsilyloxy)-2-(diethoxyphosphorylmethoxy)propoxy]-6-N-phthalimidopurine as a cream foam (1.1g, 66%). IR: $_{max}$ (film) 3070, 2930, 2860, 1795, 1603, 1580, 1470, 1450, 1430, 1405, 1365, 1330, 1250, 1175, 1160, 1105, 1020, 970, 885, 835, 795, 780 $cm^{-1}$. $^1H$ NMR: $\delta_H$ [$(CD_3)_2SO$] 0.97 (9H, s, t-Bu), 1.19, 1.20 (2x3H, 2xt, J = 7 Hz, ($OCH_2CH_3)_2$), 3.8-4.15 (9H, m, $(OCH_2CH_3)_2$, $OCH_2P$, $CHCH_2$), 4.65 (1H, dd, J = 11.5 Hz, J = 6.6 Hz, $NOCH_B$), 4.78 (1H, dd, J = 11.5 Hz, J = 3.0 Hz, $NOCH_A$), 7.3-7.7 (10H, m, 2xPh), 8.0-8.15 (4H, m, aromatic H), 9.04 (1H, s, H-2 or H-8), 9.08 (1H, s, H-2 or H-8). Found: C, 59.51; H, 5.76; N, 9.42%. $C_{37}H_{42}N_5O_8PSi$ requires: C, 59.74; H, 5.69; N, 9.41%. m/z: (FAB, thioglycerol matrix) 744 ($MH^+$).

b) A solution of (S)-9-[3-(t-butyldiphenylsilyloxy)-2-(diethoxyphosphorylmethoxy)propoxy]-6-N-phthalimidopurine (0.98g, 1.6mmol) in dry dichloromethane (10ml) was treated with N-methylhydrazine (0.093ml, 1.75mmol) at room temperature for 1 hour. The reaction mixture was filtered, the filtrate concentrated in vacuo and the residue chromatographed on silica gel using dichloromethane as the initial eluant. The eluant was changed to dichloromethane/methanol 96:4 to give (S)-9-[3-(t-butyldiphenylsilyloxy)-2-(diethoxyphosphorylmethoxy)propoxy]adenine as a colourless oil (0.61g, 75%). UV: $\lambda_{max}$ - (EtOH) 260 (13,690) nm. IR: $\nu_{max}$ (film) 3322, 3176, 2930, 2902, 2857, 1643, 1594, 1471, 1427, 1405, 1398, 1325, 1292, 1251, 1112, 1051, 1026, 971, 823, 795, 741 $cm^{-1}$. $^1H$ NMR: $\delta_H$ [$(CD_3)_2SO$] 0.94 (9H, s, t-Bu), 1.19 (2x3H, 2xt, J = 7 Hz, $(OCH_2CH_3)_2$), 3.7-4.15 (9H, m, $CHCH_2$, $OCH_2P$, $(OCH_2CH_3)_2$), 4.48 (1H, dd, J = 11.27 Hz, J = 6Hz, $NOCH_B$), 4.65 (1H, dd, J = 11.27 Hz, J = 2.75 Hz, $NOCH_A$), 7.3-7.7 (12H, m, 2xPh + $D_2O$ exchangeable $NH_2$), 8.14 (1H, s, H-2 or H-8), 8.41 (1H, s, H-2 or H-8). Found: C, 55.65; H, 6.52; N, 11.24%. $C_{29}H_{40}N_5O_6PSi$ 0.75 $H_2O$ requires: C, 55.53; H, 6.67; N, 11.16%. m/z: (FAB, thioglycerol matrix) 614 ($MH^+$).

c) (S)-9-[3-(t-Butyldiphenylsilyloxy)-2-(diethoxyphosphorylmethoxy)propoxy]adenine (0.61g, 1mmol) was dissolved in trifluoroacetic acid/water, 2:1 (6ml) and the solution stirred at ambient temperature for 3 hours. Hexane (10ml) was added and the mixture shaken. The aqueous phase was separated, washed once more with hexane (10ml) and evaporated to dryness. The residue was treated with ethanolic ammonia solution (5ml), at ambient temperature, for 5 minutes after which the solution was evaporated to dryness. The residue was chromatographed on silica gel using dichloromethane/methanol 90:10 as eluant to give (R)-9-[2-(diethoxyphosphorylmethoxy)-3-hydroxypropoxy]adenine as a colourless oil (0.3g, 80%) which on prolonged standing, crystallised as white needles m.p. 98-102°. IR: $\nu_{max}$ (film) 3340, 3210, 2995, 2930, 2910, 1660, 1645, 1600, 1470, 1445, 1415, 1395, 1370, 1330, 1300, 1240, 1210, 1165, 1125, 1045, 1020, 980, 825, 795, 735 $cm^{-1}$. $^1H$ NMR: $\delta_H$ [$(CD_3)_2SO$] 1.23 (6H, t, J = 7 Hz $(OCH_2CH_3)_2$), 3.5-3.7 (2H, m, $CH_2OH$), 3.75-3.85 (1H, m, CH), 3.95-4.15 (6H, m, $(OCH_2CH_3)_2$, $OCH_2P$), 4.38 (1H, dd, J = 11.3 Hz, J = 6.6 Hz, $NOCH_B$), 4.55 (1H, dd, J = 11.3 Hz, J = 3.0 Hz, $NOCH_A$ ), 4.88 (1H, t, J = 5.5 Hz, $D_2O$ exchangeable OH), 7.38 (2H, br.s., $D_2O$ exchangeable $NH_2$), 8.15 (1H, s, H-2 or H-8), 8.43 (1H, s, H-2 or H-8). Found: C, 39.26; H, 6.05; N, 17.33%. $C_{13}H_{22}N_5O_6P$ 1.25 $H_2O$ requires: C, 39.25; H, 6.20; N, 17.61%. m/z: (FAB, thioglycerol matrix) 376 ($MH^+$).

d) To a solution of (R)-9-[2-(diethoxyphosphorylmethoxy)-3-hydroxypropoxy)adenine (0.16g, 0.43mmol) in dry DMF (3ml) was added trimethylsilyl bromide (0.56ml, 4.3mmol) and the solution stirred at ambient temperature for 4 hours. The solvent was removed under reduced presence and the residue co-evaporated with acetone/water 1:1 (x3). The solid residue was crystallised from acetone/water (0.1g, 77%), m.p. 200-205°. UV: $\lambda_{max}$ (EtOH), 260 ( 11,800) nm. IR: $\nu_{max}$ (KBr) 3449, 3400, 3199, 3105, 2951, 2897, 1711, 1680, 1612, 1569, 1467, 1420, 1355, 1330, 1305, 1244, 1216, 1128, 1080, 924, 866 $cm^{-1}$. $^1H$ NMR: $\delta_H$ [$(CD_3)_2SO$] 3.54 (2H, m, C H $_2$OH), 3.70-3.85 (3H, m, $OCH_2P$, CH), 4.36 (1H, dd, J = 11 Hz, J = 7 Hz, $NOCH_B$), 4.55 (1H, dd, J = 11 Hz, J = 3 Hz, $NOCH_A$), 7.51 (2H, br.s, $D_2O$ exchangeable $NH_2$), 8.17 (1H, s, H-2 or H-8), 8.50 (1H, s, H-2 or H-8), 3.5-7.0 (3H, broad, $PO(OH)_2$, OH). Found: C, 32.07; H, 4.73; N, 20.46%. $C_9H_{14}N_5O_5P$ 1 $H_2O$ requires: C, 32.05; H, 4.78; N, 20.76%. m/z: (FAB, thioglycerol matrix) 320 ($MH^+$). $[\alpha]D^{25}$ (0.1M NaOH) -11.5° (c = 0.2).

Example 4

(S)-9-[3-Hydroxy-2-(phosphonomethoxy)propoxy]quanine

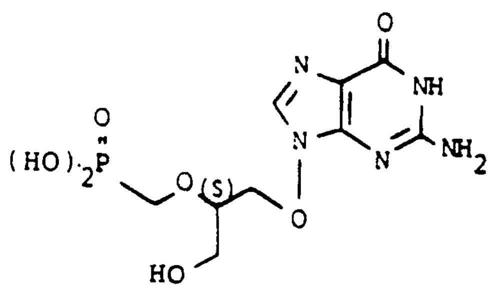

a) To a stirred solution of diethyl (R)-[2-benzyloxy-1-(hydroxymethyl)ethoxy]methylphosphonate (1.0g, 3mmol), triphenylphosphine (1.18g, 4.5mmol) and 2-[bis-(t-butoxycarbonyl)amino]-9-hydroxy-6-methoxypurine (prepared as in EP-A-319228) (1.15g, 3mmol) in dry THF (20ml), cooled in ice and under a mitrogen atmosphere, was added, dropwise, diethyl azodicarboxylate (0.71ml, 4.5mmol). The resulting solution was allowed to warm to ambient temperature and left stirring for 16 hours. The solvent was evaporated under reduced pressure and the residue obtained chromatographed on silica gel using hexane/ethyl acetate 2:1 as eluant. The eluant was changed as follows: hexane/ethyl acetate 1:1, hexane/ethyl acetate 1:2 and finally to ethyl acetate to give (S)-2-[bis-(t-butoxycarbonyl)amino]-9-[3-benzyloxy-2-(diethoxyphosphorylmethoxy)propoxy]-6-methoxypurine as a colourless oil (1.85g, 88%).
IR: $_{max}$ (film) 3070, 2990, 2940, 2910, 1795, 1755, 1740, 1595, 1480, 1455, 1425, 1395, 1370, 1325, 1255, 1160, 1120, 1100, 1050, 1025, 975, 855, 795, 740, 705$cm^{-1}$. $^1H$ NMR: $\delta_H$[$(CD_3)_2SO$] 1.20 (6H, t, J = 7 Hz, ($OCH_2C$ H $_3)_2$), 1.38 (18H, s, 2xt-Bu), 3.5-3.75 (2H, m, $CH_2$), 3.95-4.1 (7H, m, $OCH_2P$, CH, (OC H $_2CH_3)_2$), 4.08 (3H, s, $OCH_3$), 4.45-4.65 (2H, m, $NOCH_2$) 4.51 (2H, s, OC H $_2$Ph), 7.25-7.40 (5H, m, Ph), 8.75 (1H, s, H-8). Found: C, 52.27; H, 6.77; N, 9.65%. $C_{31}H_{45}N_5O_{11}P$ 1$H_2O$ requires: C, 52.17; H, 6.77; N, 9.81%. m/z: (FAB, TDE/Na matrix) 718 (32%, $M+Na^+$) 696 (13%, $MH^+$).
b) Trifluoroacetic acid (2ml) was added to an ice cooled solution of (S)-2-[bis-(t-butoxycarbonyl)amino]-9-[3-benzyloxy-2-(diethoxyphosphorylmethoxy]propoxy]-6-methoxypurine (1.65g, 2.3mmol) in dry dichloromethane (30ml) and the resulting solution left for 2 hours. After warming to ambient temperature, 10% Pd-C (800mg) was added and the mixture hydrogenated at ambient temperature and pressure for 2 hours. The reaction mixture was filtered, the catalyst washed with dichloromethane (20ml), the filtrate washed with saturated aqueous sodium bicarbonate solution, brine, dried ($MgSO_4$) and evaporated to leave a colourless viscous oil. Chromatography of the oil on silica gel using dichloromethane/methanol 95:5 as the initial eluant, then changing the eluant to dichloromethane/methanol 90:10 gave (S)-2-amino-9-[2-(diethoxyphosphorylmethoxy)-3-hydroxypropoxy]-6-methoxypurine as a colourless oil (0.65g, 67%).
IR: $\nu_{max}$ (film) 3360, 3230, 2990, 2940, 2910, 1620, 1585, 1500, 1485, 1455, 1390, 1335, 1265, 1240, 1165, 1120, 1060, 1025, 975, 825, 785$cm^{-1}$. $^1H$ NMR: $\delta_H$ [$(CD_3)_2SO$] 1.23 (6H, t, J = 7 Hz, ($OCH_2C$ H $_3)_2$), 3.45-3.60 (2H, m, collapses to d on $D_2O$ exchange, C H $_2$OH), 3.70-3.85 (1H, m, CH), 3.96 (3H, s, $OCH_3$), 3.9-4.15 (6H, m, (OC H $_2CH_3$, $OCH_2P$), 4.31 (1H, dd, J = 11.3 Hz, J = 6.9 Hz, $NOCH_B$), 4.45 (1H, dd, J = 11.3 Hz, J = 3 Hz, $NOCH_A$), 4.84 (1H, t, J = 5.5 Hz, $D_2O$ exchangeable OH), 6.59 (2H, br.s, $D_2O$ exchangeable $NH_2$), 8.14 (3H, s, H-8). m/z: (FAB, thioglycerol matrix) 406 ($MH^+$).
c) To a solution of (S)-2-amino-9-[2-(diethoxyphosphorylmethoxy)-3-hydroxypropoxy]-6-methoxypurine (0.56g, 1.38mmol) in dry dimethylformamide (5ml) was added trimethylsilyl bromide (1.82ml, 13.8mmol) and the solution stirred at ambient temperature for 3 hours. The solvent was removed under reduced

pressure and the residue co-evaporated with acetone/water 1 : 1 (x2). The white solid obtained was crystallised from hot water to give the title compound as a white solid (0.305g, 66%).
IR:$\nu_{max}$ (KBr) 3380, 3320, 3160, 1715, 1645, 1600, 1385, 1185, 1160, 1100, 1060, 1045, 925, 770cm$^{-1}$. $^1$H NMR: $\delta_H$ ($D_2O$ + $NH_3$) 3.61 (1H, dd, J = 12.1 Hz J = 9.9 Hz, $CH_BP$), 3.65-3.75 (2H, m, $CH_AP$, C H $_BOH$), 3.82-3.89 (1H, m, CH), 3.93 (1H, dd, J = 12.3 Hz, J = 3.6 Hz, C H $_AOH$), 4.46-4.53 (2H, m, $NOCH_2$), 8.03 (1H, s, H-8). Found: C, 32.24; H, 4.16; N, 20.59%. $C_9H_{14}N_5O_7P$ requires: C, 32.24; H, 4.21; N, 20.89%. m/z: (FAB, thioglycerol matrix) 336 ($MH^+$). $[\alpha]D^{25}$ (0.1M NaOH) + 12.8° (c = 0.43).

Example 5

(R)-9-[(2-Hydroxy-2-oxo-1,4,2-dioxaphosphorinan-5-yl)methoxy]quanine

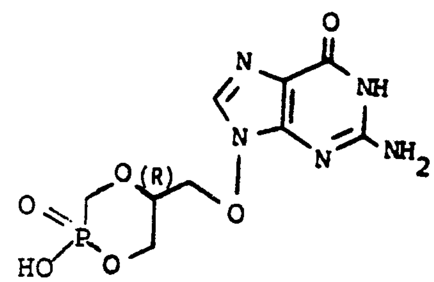

A solution of dicyclohexylcarbodiimide (0.615g, 3.0mmol) in t-butanol (10ml) was added dropwise over 30 minutes to a solution of (S)-9-[3-hydroxy-2-(phosphonomethoxy)propoxy]guanine (0.20g, 0.6mmol) and N,N-dicyclohexyl-4-morpholinocarboxamidine (0.175g, 0.6mmol) in 50% aqueous t-butanol (30ml). The reaction mixture was heated under reflux for 4 hours, allowed to cool to ambient temperature and evaporated to dryness. Water was added to the residue and the mixture filtered through a glass fibre filter paper. The filtrate was evaporated to dryness and the solid obtained purified on DEAE-Sephadex ($HCO_3$ form) eluting with a linear gradient of aqueous triethylammonium bicarbonate (pH 7.5) from 0.001M to 0.25M. Relevant fractions were combined and evaporated to dryness co-evaporating with ethanol/water, 3:1 (x2) to remove all traces of triethylamine. The product was converted into the sodium salt using DOWEX 50W-X8 ($Na^+$ form). The title compound was obtained as a white solid (0.12g, 59%) after lyophilisation. IR: $\nu_{max}$ (KBr) 3400, 3140, 1690, 1610, 1475, 1380, 1240, 1208, 1079, 1040, 1010, 960, 790cm$^{-1}$. $^1$H NMR: $\delta_H$ [-$(CD_3)_2SO$] 3.42 (1H, dd, J = 13.2 Hz, J = 3.3 Hz, $OCH_BP$), 3.55 (1H, dd, J = 13.2 Hz, J = 8 Hz, $OCH_AP$), 3.79 (1H, m, CH), 3.92 (1H, m, $CH_BOP$), 4.07-4.32 (3H, m, $CH_AOP$, $NOCH_2$), 6.86 (2H, br.s, $D_2O$ exchangeable $NH_2$), 7.90 (1H, s, H-8), 10.97 (1H, br.s, $D_2O$ exchangeable NH). m/z: (FAB, thioglycerol) 318 (M-Na+2$H^+$), 340 ($MH^+$).

Example 6

(S)-9-[3-Hydroxy-2-(phosphonomethoxy)propoxy]adenine

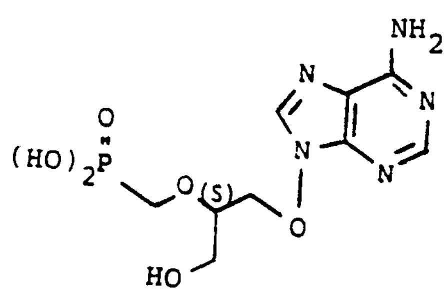

a) To a stirred solution of diethyl (R)-[2-benzyloxy-1-(hydroxymethyl)ethoxy]methylphosphonate (0.9g, 2.7mmol), triphenylphosphine (1.06g, 4mmol) and 9-hydroxy-6-N-phthalimidopurine (0.76g, 2.7mmol) (prepared as in EP-A-319228) in dry THF (15ml), cooled in ice and under a nitrogen atmosphere, was added, dropwise, diethyl azodicarboxylate (0.6ml, 4mmol). The resulting solution was allowed to warm to ambient temperature and stirred for 4 hours. The solvent was evaporated under reduced pressure and the residue obtained chromatographed on silica gel using hexane/ethyl acetate 1:1 as the initial eluant. The eluant was changed to ethyl acetate to give (S)-9-[3-benzyloxy-2-(diethoxyphosphorylmethoxy)-propoxy]-6-N-phthalimidopurine as a pale brown oil (1.16g, 72%) which was used directly in the next stage.

b) A solution of (S)-9-[3-benzyloxy-2-(diethoxyphosphorylmethoxy)propoxy]-6-N-phthalimidopurine (0.83g, 1.39mmol) in dry dichloromethane (10ml) was treated with N-methylhydrazine (0.08ml, 1.53mmol) at ambient temperature for 3 hours. The reaction mixture was filtered, the filtrate concentrated in vacuo and the residue chromatographed on silica gel using dichloromethane/methanol 98:2 as eluant to give (S)-9-[3-benzyloxy-2-(diethoxyphosphorylmethoxy)-propoxy]adenine as a colourless gum (0.27g, 41%).

IR: $\nu_{max}$ (film) 3320, 3180, 2990, 2905, 1650, 1595, 1470, 1455, 1410, 1390, 1370, 1295, 1245, 1160, 1095, 1050, 1025, 970, 820, 790, 730, 700cm$^{-1}$. $^1$H NMR: $\delta_H$ [$(CD_3)_2SO$] 1.21 (2x3H, 2xt, J = 7Hz, ($OCH_2C\underline{H}_3)_2$), 3.61-3.72 (2H, m, $C\underline{H}_2OCH_2Ph$), 3.96-4.12 (7H, m, CH, $OCH_2P$, ($OC\underline{H}_2CH_3)_2$), 4.40-4.58 (2H, m, $NOCH_2$), 4.52 (2H, s, $OCH_2Ph$), 7.25-7.50 (7H, m, Ph + $D_2O$ exchangeable $NH_2$), 8.15 (1H, s, H-2 or H-8), 8.41 (1H, s, H-2 or H-8).

c) 10% Pd-C (50mg) was added to a solution of (S)-9-[3-benzyloxy-2-(diethoxyphosphorylmethoxy)-propoxy]adenine (0.21g, 0.45mmol) in dichloromethane (5ml) and trifluoroacetic acid (1ml) and the mixture hydrogenated at atmospheric pressure and ambient temperature for 5 hours. The mixture was filtered, the filtrate evaporated and the residue obtained dissolved in ethanolic ammonia solution (5ml). After 15 minutes the solvent was evaporated and the residue chromatographed on silica gel using dichloromethane/methanol 90:10 as eluant. T.l.c. examination of the product indicated traces of a non UV absorbing impurity which was removed by prep. t.l.c. using dichloromethane/methanol 88:12 as eluant. (S)-9-[2-(diethoxyphosphorylmethoxy)-3-hydroxypropoxy]adenine was obtained as a colourless gum (0.11g, 65%).

$^1$H NMR: $\delta_H$[$(CD_3)_2SO$] 1.23 (6H, t, J = 7Hz, ($OCH_2C\underline{H}_3)_2$), 3.5-3.65 (2H, m, $C\underline{H}_2OH$), 3.75-3.85 (1H, m, CH), 3.98-4.15 (6H, m, $OCH_2P$, ($OC\underline{H}_2CH_3)_2$), 4.38 (1H, dd, J = 6.6Hz, J = 11.3Hz, $NOCH_B$), 4.54 (1H, dd, J = 3Hz, J = 11.3Hz, $NOCH_A$), 4.88 (1H, t, J = 5.5Hz, $D_2O$ exchangeable OH), 7.38 (2H, br.s, $D_2O$ exchangeable $NH_2$), 8.15 (1H, s, H-2 or H-8), 8.43 (1H, s, H-2 or H-8). m/z: (FAB+, thioglycerol matrix) 376 ($MH^+$).

d) To a solution of (S)-9-[2-(diethoxyphosphorylmethoxy)-3-hydroxypropoxy]adenine(0.09g, 0.24mmol) in dry dichloromethane (3ml) was added trimethylsilyl bromide (0.31ml, 2.4mmol) and the solution stirred at ambient temperature for 4 hours. The solvent was removed under reduced pressure and the residue co-evaporated with acetone/water 1:1 (x3). Crystallisation of the residue from water/acetone gave the title compound as a white solid (0.06g, 78%), mp. 193-198°C.

$^1$H NMR: $\delta_H$ [$(CD_3)_2SO$] 3.55 (2H, m, $C\underline{H}_2OH$), 3.65-3.85 (3H, m, $OCH_2P$, CH), 4.36 (1H, dd, J = 7Hz, J = 11Hz, $NOCH_B$), 4.55 (1H, dd, J = 3Hz, J = 11Hz, $NOCH_A$), 7.61 (2H, br.s, $D_2O$ exchangeable $NH_2$), 8.19 (1H, s, H-2 or H-8), 8.52 (1H, s, H-2 or H-8), 2.7-5.5 (3H, broad, $D_2O$ exchangeable $PO(OH)_2$, OH).

The following (R)- and (S)-isomers of the compounds of formula (IA) are prepared analogously according to methods hereinbefore described.

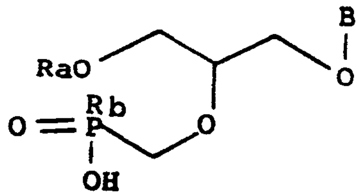

| B | Ra | Rb |
|---|---|---|
| 2,4-diaminopurine | H | OH |
| adenine | a bond | |
| 2,4-diaminopurine | a bond | |

Antiviral Activity

1. Plaque Reduction Test for Herpes Simplex Viruses 1 and 2

Vero or MRC-5 cells were grown to confluence in 24 well multi-dishes (well diameter = 1.5cm). The drained cell monolayers were each infected with approximately 50 infectious particles of herpes simplex virus 1 (HSV-1; HFEM strain) or herpes simplex virus 2 (HSV-2; strain MS) in 100µl of phosphate-buffered saline. The virus was adsorbed for 1 hour at room temperature. After adsorption, residual inoculum was removed from each well and replaced with 0.5ml of Eagle's MEM containing 5% newborn calf serum and 0.9% agarose (A37). Once the agarose had set, dilutions of the test compound, which had been prepared in Eagle's MEM (containing 5% newborn calf serum), were added, each well receiving 0.5ml of liquid overlay. The test compound was diluted to give the following series of concentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (2 or 3 days for Vero cells, usually 1 day for MRC-5 cells).

2. Plaque Reduction Test for Varicella Zoster Virus

MRC-5 cells were grown to confluence in 24 well multi-dishes (well diameter = 1.5cm). The drained cell monolayers were each infected with approximately 50 infectious particles of varicella zoster virus (VZV; Ellen strain) in 100µl of phosphate-buffered saline. The virus was adsorbed for 1 hour at room temperature. After adsorption, residual inoculum was removed from each well and replaced with 0.5ml of Eagle's MEM containing 5% heat-inactivated foetal calf serum and 0.9% agarose (A37). Once the agarose had set, dilutions of the test compound, which had been prepared in Eagle's MEM (containing 5% heat-inactivated foetal calf serum), were added, each well receiving 0.5ml of liquid overlay. The test compound was diluted to give the following series of concentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (5 or 6 days).

Cultures from 1 and 2 were fixed in formal saline, the agarose overlays were carefully washed off, and then the cell monolayers were stained with carbol fuchsin. A stereo microscope was used to count plaques. The $IC_{50}$ (concentration of drug which inhibits the number of plaques formed by 50% relative to the number of plaques observed in virus control monolayers) of the test compound was calculated. In addition, the monolayers were examined for evidence of drug-induced cytotoxicity; the minimum concentration at which cytotoxicity occurred was recorded.

3. Plaque Reduction Test for Cytomegalovirus

MRC-5 cells were grown to confluence in 24 well multi-dishes (well diameter = 1.5cm). The drained cell monolayers were each infected with approximately 50 infectious particles of cytomegalovirus (CMV; AD-169 strain) in 100µl of phosphate-buffered saline. The virus wasadsorbed for 1 hour at room temperature. After adsorption, residual inoculum was removed from each well and replaced with 1ml of Eagle's MEM containing 10% heatinactivated foetal calf serum and 0.9% agarose (A37). Once the agarose had set, dilutions of the test compound, which had been prepared in Eagle's MEM (containing 10% heat-inactivated calf serum), wereadded, each well receiving 1ml of liquid overlay. The test compound was diluted to give the following series of concentrations: 200, 60, 20, 6....0.06µg/ml; final concentrations in the assay range, therefore, between 100µg/ml and 0.03µg/ml. The infected cultures were incubated at 37°C in a humidified atmosphere containing 5% $CO_2$ until plaques were clearly visible (about 12days). The cultures are fixed in formol saline, the agarose overlays were carefully washed off, and then the cell monolayers were stained with carbol fuchsin. A stereo microscope was used to count plaques. The $IC_{50}$ (concentration of drug which inhibits the number of plaques formed by 50% relative to the number of plaques observed in virus control monolayers) of the test compound was calculated. In addition, the monolayers were examined for evidence of drug-induced cytotoxicity; the minimum concentration at which cytotoxicity occurs was recorded.

4. Test for Human Immunodeficiency Virus (HIV)

a) Cell cytotoxicity test

Peripheral human lymphocytes were isolated by density gradient centrifugation from blood donations of healthy volunteers. The 'buffy coat' fractions of these donations were provided by blood donation centres.

The buffy coat was diluted 1:1 with sterile phosphate buffered saline (PBS; 50 mM sodium phosphate, pH 7.4, 0,9% sodium chloride) and subsequently layered over Ficoll. Following centrifugation (30 minutes at 400 x g) the supernatant was discarded and the interphase containing the lymphocytes was recovered. The lymphocytes were washed two times in PBS and were resuspended finally in cell culture medium.

A viability staining was performed by means of the trypan blue dye-exclusion method. The concentration of cells in the suspension and the percentage of viable cells were calculated. Subsequently, the cell suspension was adjusted to a concentration of $1x10^7$ cells/ml. This cell suspension was transferred to tissue culture flasks: Two thirds of the cell suspension were polyclonally activated by addition of phytohemagglutinin (final concentration 5 µg.ml). One third of the cell suspension remained unstimulated.

The lymphocytes were cultivated in an incubator with a humidified atmosphere and 5% $CO_2$ for 48 to 64 hours at 37°C. Following this incubation period, cells were harvested by centrifugation, resuspended in cell culture medium and counted. Stimulated and unstimulated cells were combined in a ratio of 2:1 and adjusted to a concentration of $2x10^5$ cells/ml with cell culture medium that contained, in addition, 10 units/ml of human recombinant interleukin-2.

Only those preparations of lymphocytes were employed for the screening test in which more than 70% of the stimulated lymphocytes expressed the CD 25 antigen and more than 45% of the lymphocytes expressed the CD 4 antigen.

100µg of this lymphocyte suspension was added to each well of microtiter plates containing the test compounds serially diluted over the range 100 µM to 0.1µM. Subsequently, the microtiter plates were cultivated for 4 days at 37°C.

Survival and proliferation of the lymphocytes grown in the presence of the compound were measured by a quantitative colorimetric assay. Viable cells cultivated in the presence of the dye MTT [3-4,5-dimethylthiazol-2-yl)-3,5-diphenyltetrazolium) reduce this pale yellow substrate by activity of their mitochondrial dehydrogenases to a purple formazan. The amount of product which is a function of cell number and metabolic cellular activity was quantified photometrically. By this assay, potential cytotoxic and cytostatic effects of compounds towards lymphocytes were detected precisely.

Microtiter plates were centrifuged for 5 minutes at 900 x g. The supernatant was discarded and the cells of each well were resuspended in 50 µl of cell culture medium containing 2mg/ml of MTT. After four hours of incubation at 37°C 100 µl of solvent (isopropanol with 0,04 N HCl and 10% (v/v) Triton 100) was added to each well. By shaking the microtiter plates the formazan was solubilized. Subsequently, the plates were evaluated in an ELISA photometer in the dual wavelength mode (measuring wavelength: 550 nm; reference wavelength: 690 nm).

For each well the difference in absorption (abs. 550 nm - abs. 690 nm) was calculated. These data provided the basis for further evaluation of the cytotoxicity test. The approximate $CD_{50}$ (halfmaximal

cytotoxic dose) of each compound was calculated.

b) HIV Suppression test

Peripheral human lymphocytes were prepared, cultivated, and harvested as above. Following the determination of the lymphocyte surface markers, unstimulated and mitogen stimulated cells were combined in a ratio of 1:2.

Under safety conditions these cells are infected with a standard preparation of HIV. The cells are sedimented by centrifugation. The supernatant was discarded and cells were resuspended in the HIV inoculum.

This inoculum is a liquid suspension of HIV-1 strain virus, pretested and adjusted to a titer that results in a synthesis of viral core protein p24 of >100 ng/ml at day four following infection of human lymphocytes according to the protocol.

$3x10^8$ lymphocytes were resuspended in 1 ml HIV inoculum and incubated at 37°C for 60 minutes. Subsequently, the cells were washed two times with 50 ml of culture medium and resuspended in culture medium containing 10 units/ml of human recombinant interleukin-2 to yield a cell concentration of $2x10^6$ cells/ml. 100µl of this cell suspension was added to each well of the microtiter plates containing the diluted solutions of the compounds. The microtiter plates were cultivated in an incubator with a humidified atmosphere and 5% $CO_2$ at 37°C.

Accordingly, a fraction of lymphocytes was mock-infected with the same virus preparation that was heat inactivated (30 minutes at 56°C) prior to infection.

On each of the days 2,3 and 4 post infection one of the microtiter plates which had been established in triplicate was prepared for determination of viral replication. Viral RNA is determined within the cells whereas the viral core protein p24 was detected in the supernatant of the lymphocyte culture.

Accordingly, 150 µl of supernatant were removed from each well and transferred to the well of a microtiter plate containing 50 µl/well of SDS (sodium dodecylsulfate, 0.08%). These plates were stored frozen. 50 µl of stop solution (1% SDS, 20mM sodium acetate, pH 5.0, and 200 µg/ml heparin) were added to the cells remaining in each well. The plates were stored frozen.

The concentration of p24 synthesized by the HIV infected cells was determined by means of a sandwich ELISA, while the concentration of viral RNA was quantitated by nucleic acid hybridisation, using a $^{32}P$-labelled DNA probe for the gag/pol region of the viral genome. Absolute levels of viral antigen and RNA in drug treated samples were compared with untreated, virus-infected controls and the percentage inhibition calculated.

Results

The compounds of Examples 1, 2, 4 and 5 were active against HSV-1 virus with $IC_{50}$ values of 7, 21, 25 and 73 µg/ml respectively. The same compounds tested against HSV-2 gave $IC_{50}$ values of 10, 15, 23 and 36 µg/ml respectively and against VZV gave 17, 13, 52 and 24 mg/ml respectively and against CMV gave 3, 3, 29 and 17 ug/ml respectively.

gainst HIV virus, the following results were obtained.

| | | % Inhibition on Days 3 and 4 after infection | | | |
|---|---|---|---|---|---|
| Ex. No. | Concn. (µM) | Viral Anitgen | | Viral RNA | |
| | | Day 3 | Day 4 | Day 3 | Day 4 |
| 1 | 1 | 100 | 35 | 73 | 54 |
| 2 | 10 | 100 | 33 | 92 | 32 |
| 3 | 10 | 80 | 91 | 83 | 90 |
| 4 | 1 | 54 | 15 | 10 | 14 |
| 5 | 10 | 50 | 7 | 64 | 0 |

## Claims

1. The (R)-isomer or the (S)-isomer of a compound of formula (IA), optionally in admixture with up to 45% of the mixture by weight of the corresponding (S)-isomer or the (R)-isomer respectively:

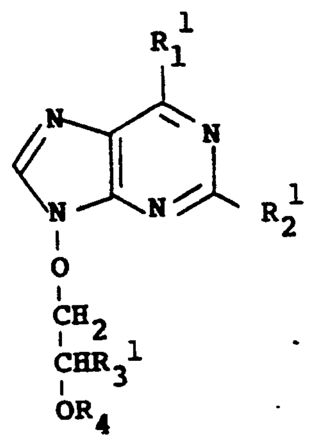

(IA)

wherein
$R_1^1$ is hydroxy or amino;
$R_2^1$ is amino or hydrogen;
$R_3^1$ is hydroxymethyl or acyloxymethyl; and
$R_4$ is a group of formula:

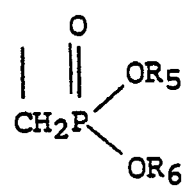

wherein
$R_5$ and $R_6$ are independently selected from hydrogen, $C_{1-6}$ alkyl and optionally substituted phenyl; or
$R_3$ and $R_4$ together are:

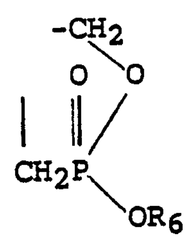

wherein
$R_6$ is as defined above.

2. A compound according to claim 1 wherein $R_1^1$ is hydroxy and $R_2^1$ is amino.
3. A compound according to claim 1 wherein $R_1^1$ is amino and $R_2^1$ is hydrogen.
4. A compound according to any one of claims 1 to 3 wherein $R_3^1$ is hydroxymethyl.
5. A compound according to any one of claims 1 to 4 wherein $R_5$ and $R_6$ are both hydrogen.
6. An isomer according to any one of claims 1 to 5, which is the (R)-isomer when $R_3$ and $R_4$ are not joined and the (S)-isomer when $R_3$ and $R_4$ are joined.
7. An (R)-isomer or (S)-isomer according to any one of claims 1 to 6, in a form containing from 0 to 5% of the corresponding (S)-isomer or the (R)-isomer respectively.
8. An isomer according to claim 1 or 7 which is (R)-9-[3-hydroxy-2-(phosphonomethoxy)propoxy]guanine, (S)-9-[(2-hydroxy-2-oxo-1,4,2-dioxaphosphorinan-5-yl)methoxy]guanine,

(R)-9-[3-hydroxy-2-(phosphonomethoxy)propoxy]adenine,
(S)-9-[3-hydroxy-2-(phosphonomethoxy)propoxy]guanine,
(R)-9-[(2-hydroxy-2-oxo-1,4,2-dioxaphosphorinan-5-yl)methoxy]guanine, or
(S)-9-[3-hydroxy-2-(phosphonomethoxy)propoxy]adenine.

9. A process for the preparation of an isomer according to claim 1, which process comprises either

i) imidazole ring closure of a compound of formula (II):

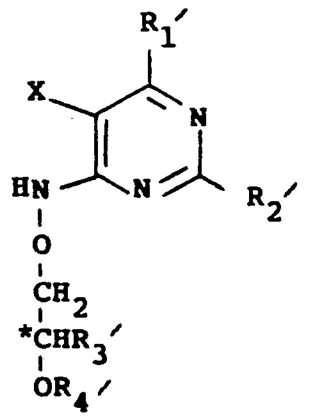

(II)

wherein X is a group capable of cyclising to form an imidazole ring, such as amino or an amino derivative, for example, formylamino; or

ii) pyrimidine ring closure of a compound of formula (III):

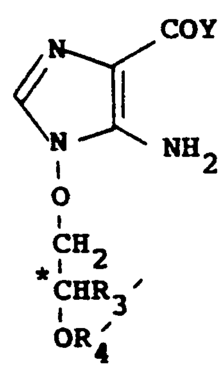

(III)

wherein Y is amino or $C_{1-6}$ alkoxy, with a condensing agent capable of cyclising to form a pyrimidine ring having a 2-$R_2'$ substituent, to give a compound of formula (I) wherein $R_1$ is hydroxy and $R_2$ is amino; or

iii) condensing a compound of formula (IV):

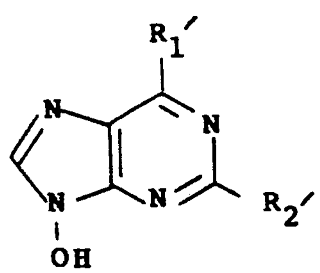

(IV)

with a side chain intermediate of formula (V):

$ZCH_2$- $\overset{*}{C}HR_3'OR_4'$ (V)

wherein Z is a leaving group and the carbon atom marked "*" in formulae (II), (III) and (V) is 55% or more in the configuration resulting in the (R)-isomer or the (S)-isomer of a compound of formula (IA); and wherein, in formulae (II) to (V), $R_1'$, $R_2'$, $R_3'$, $R_4'$ are $R_1^1$, $R_2^1$, $R_3^1$ and $R_4$ respectively, or groups or atoms convertible thereto; and thereafter, when desired or necessary, converting $R_1'$, $R_2'$, $R_3'$ and/or $R_4'$, when other than $R_1^1$, $R_2^1$, $R_3^1$ and/or $R_4$ to $R_1^1$, $R_2^1$, $R_3^1$ and/or $R_4$ respectively, and/or converting $R_1'$, $R_2'$, $R_3'$ and/or $R_4'$ when $R_1^1$, $R_2^1$, $R_3^1$ and/or $R_4$, to other $R_1^1$, $R_2^1$, $R_3^1$ and/or $R_4$.

10. A pharmaceutical composition comprising a compound according to any one of claims 1 to 8, and a pharmaceutically acceptable carrier.

11. A compound according to any one of claims 1 to 8 for use as an active therapeutic substance.

12. A compound according to any one claims 1 to 8 for use in treating viral infections or in treating neoplastic diseases.

13. Use of a compound according to any one of claims 1 to 8 in the manufacture of a medicament for use in the treatment of viral infections or neoplastic diseases.

Claims for the following Contracting State: ES

1. A process for the preparation of the (R)-isomer or the (S)-isomer of a compound of formula (IA), optionally in admixture with up to 45% of the mixture by weight of the corresponding (S)-isomer or the (R)-isomer respectively:

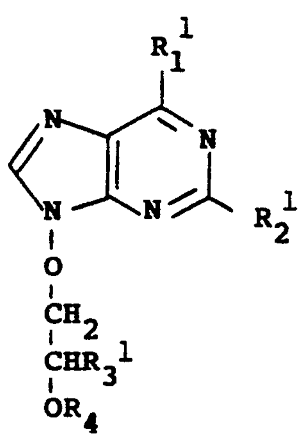

(IA)

wherein

$R_1^1$ is hydroxy or amino;

$R_2^1$ is amino or hydrogen;

$R_3^1$ is hydroxymethyl or acyloxymethyl; and

$R_4$ is a group of formula:

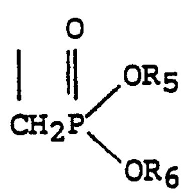

wherein

$R_5$ and $R_6$ are independently selected from hydrogen, $C_{1-6}$ alkyl and optionally substituted phenyl; or

$R_3$ and $R_4$ together are:

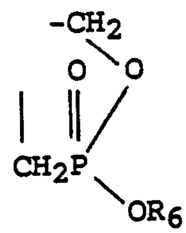

wherein
$R_6$ is as defined above;
which process comprises either
i) imidazole ring closure of a compound of formula (II):

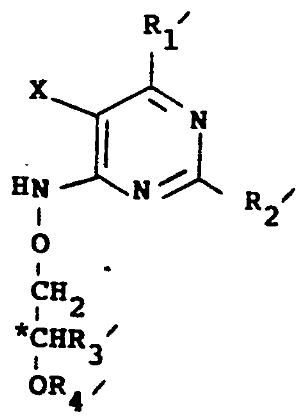

(II)

wherein X is a group capable of cyclising to form an imidazole ring, such as amino or an amino derivative, for example, formylamino; or
ii) pyrimidine ring closure of a compound of formula (III):

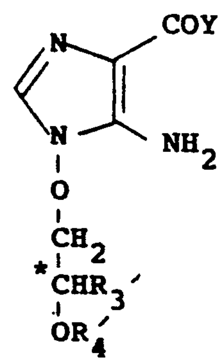

(III)

wherein Y is amino or $C_{1-6}$ alkoxy, with a condensing agent capable of cyclising to form a pyrimidine ring having a 2-$R_2'$ substituent, to give a compound of formula (I) wherein $R_1$ is hydroxy and $R_2$ is amino; or
iii) condensing a compound of formula (IV):

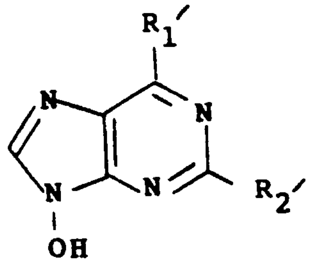

(IV)

with a side chain intermediate of formula (V):

$ZCH_2$- $\overset{*}{C}HR_3'OR_4'$ (V)

wherein Z is a leaving group and the carbon atom marked "*" in formulae (II), (III) and (V) is 55% or more in the configuration resulting in the (R)-isomer or the (S)-isomer of a compound of formula (IA); and wherein, in formulae (II) to (V), $R_1'$, $R_2'$, $R_3'$, $R_4'$ are $R_1^1$, $R_2^1$, $R_3^1$ and $R_4$ respectively, or groups or atoms convertible thereto; and thereafter, when desired or necessary, converting $R_1'$, $R_2'$, $R_3'$ and/or $R_4'$, when other than $R_1^1$, $R_2^1$, $R_3^1$ and/or $R_4$ to $R_1^1$, $R_2^1$, $R_3^1$ and/or $R_4$ respectively, and/or converting $R_1'$, $R_2'$, $R_3'$ and/or $R_4'$ when $R_1^1$, $R_2^1$, $R_3^1$ and/or $R_4$, to other $R_1^1$, $R_2^1$, $R_3^1$ and/or $R_4$.

2. A process for the preparation of the (R)-isomer or the (S)-isomer of a compound of formula (IA) as defined in claim 1, or a pharmaceutically acceptable salt thereof, optionally in admixture with up to 45% of the mixture by weight of the corresponding (S)-isomer or the (R)-isomer respectively, which process comprises resolution of a mixture of (R)- and (S)-isomers of a compound of formula (IA) to the extent that the resolution fraction which predominantly contains (R)-isomer or the (S)-isomer optionally contains up to 45% of that resolution fraction by weight of the corresponding (S)-isomer or the (R)-isomer respectively; and optionally forming a pharmaceutically acceptable salt thereof.

3. A process according to claim 1 or 2 wherein $R_1^1$ is hydroxy and $R_2^1$ is amino.

4. A process according to claim 1 or 2 wherein $R_1^1$ is amino and $R_2^1$ is hydrogen.

5. A process according to any one of claims 1 to 4 wherein $R_3^1$ is hydroxymethyl.

6. A process according to any one of claims 1 to 5 wherein $R_5$ and $R_6$ are both hydrogen.

7. A process for according to claim 1 or 2 for the preparation of an isomer as defined in any one of claims 1 to 5, which is the (R)-isomer when $R_3$ and $R_4$ are not joined and the (S)-isomer when $R_3$ and $R_4$ are joined.

8. A process according to claim 1 or 2 for the preparation of an (R)-isomer or (S)-isomer as defined in any one of claims 1 to 6, in a form containing from 0 to 5% of the corresponding (S)-isomer or the (R)-isomer respectively.

9. A process according to claim 1 or 2 for the preparation of an isomer as defined in claim 1 or 7 which is
(R)-9-[3-hydroxy-2-(phosphonomethoxy)propoxy]guanine,
(S)-9-[(2-hydroxy-2-oxo-1,4,2-dioxaphosphorinan-5-yl)methoxy]guanine,
(R)-9-[3-hydroxy-2-(phosphonomethoxy)propoxy]adenine,
(S)-9-[3-hydroxy-2-(phosphonomethoxy)propoxy]guanine,
(R)-9-[(2-hydroxy-2-oxo-1,4,2-dioxaphosphorinan-5-yl)methoxy]guanine, or
(S)-9-[3-hydroxy-2-(phosphonomethoxy)propoxy]adenine.

10. Use of an isomer as defined in claim 1, in the manufacture of a medicament for use in the treatment of viral infections or neoplastic diseases.

European Patent Office

# EUROPEAN SEARCH REPORT

Application number

EP 90305689.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| P,D, A | EP - A2 - 0 319 228 (BEECHAM) * Abstract; claims * -- | 1, 10-13 | C 07 F 9/38<br>C 07 F 9/6574<br>A 61 K 31/66 |
| A | EP - A2 - 0 313 289 (BEECHAM) * Abstract * ---- | 1 | |
| | | | TECHNICAL FIELDS SEARCHED (Int Cl 5) |
| | | | C 07 F<br>A 61 K |

The present search report has been drawn up for all claims

| Place of search | Date of completion of the search | Examiner |
|---|---|---|
| VIENNA | 27-08-1990 | MARCHART |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82